# EUROPEAN PATENT SPECIFICATION

(11) **EP 2 197 504 B1**
(45) Date of publication and mention of the grant of the patent: **31.08.2011**
(21) Application number: 08788520.8
(22) Date of filing: 03.09.2008
(51) Int. Cl.: A61L 27/24, A61L 27/46

(54) **BIOMATERIAL SCAFFOLDS FOR CONTROLLED TISSUE GROWTH**
GERÜSTE AUS BIOLOGISCHEM MATERIAL FÜR KONTROLLIERTES GEWEBEWACHSTUM
ÉCHAFAUDAGES EN BIOMATÉRIAU UTILISÉS POUR LA CROISSANCE TISSULAIRE RÉGULÉE

(30) Priority: 04.09.2007 GB 0717168
(43) Date of publication of application: 23.06.2010
(73) Proprietor: UCL Business PLC, London W1T 4TP (GB)
(72) Inventor: BROWN, Robert, Stanmore Middlesex HA7 4LP (GB); ALEKSEEVA, Tijna, London WD23 2NE (GB)
(74) Representative: Sutcliffe, Nicholas Robert
(86) International application number: PCT/GB2008/002982
(87) International publication number: WO 2009/030901

(56) References cited:
- WO-A-2005/046457
- WO-A-2007/056418
- NAZHAT SHOWAN N ET AL: "Controlled microchannelling in dense collagen scaffolds by soluble phosphate glass fibers" BIOMACROMOLECULES, ACS, WASHINGTON, DC, US, vol. 8, 1 January 2007 (2007-01-01), pages 543-551, XP007909602 ISSN: 1525-7797 [retrieved on 2009-01-01]

## Description

This invention relates to implants and tissue scaffolds which support directional and/or timed cell and/or tissue growth, for example for use in the repair of damaged tissues and the promotion of angiogenesis, innervation or re-nervation, and/or mechanical integration (adhesion) across biological interfaces (e.g. at a host-implant interface).

The maintenance of seeded cell viability and function is problematic in tissue engineering scaffolds because of limitations in oxygen and nutrient transfer.

One approach to the maintenance of suitable oxygen concentrations throughout a tissue-engineering scaffold would be to controllably incorporate microchannelling within the scaffold as part of the fabrication process at the time of fabrication. The formation of micro-channels in aqueous-based tissue constructs and scaffolds in a precise engineered manner using soluble (phosphate) glass (SG) fibres of known diameter, spacing/density and degradation rate has been reported (Nazhat et al 2007(1) Biomolecules (2007) Feb;8(2):543-51). The soluble glass fibres were incorporated into a scaffold 3D structure in the pattern required for eventual micro vascular channelling and subsequently, the fibres dissolved leaving channels in the size and position of the original fibres.

Directional-timed in-growth would be useful for optimal tissue regeneration in tissue engineering scaffolds, for example, in nerve (peripheral or spinal) regeneration where axons and support cells need to connect between 2 points, with an "in" and "out" direction. Known techniques for such directional-timed in-growth involve molecular gradients of growth factors and are inherently non-controlled and unsuitable for tissue engineering scaffolds, especially in three dimensions and over significant distances (e.g. >1mm).

The present invention relates to the use of soluble fibres having a variable cross-sectional area to control the direction and rate of formation of microchannels in a biomaterial. The soluble fibre dissolves in a directional manner thereby allowing vectored channelling through the biomaterial. Furthermore, the fibre preserves the space required by regenerating tissue until the appropriate tissue reaches it, thereby allowing timed channelling through the biomaterial.

This may be useful, for example in directing the growth of blood vessels, nerves and other repair (integration) cells through the biomaterial in a particular direction (i.e. directional growth).

An aspect of the invention provides a biomaterial for directional tissue growth comprising;
a soluble fibre disposed within a gel,
the fibre having tissue entry and tissue exit ends,
wherein the cross-sectional area of the fibre progressively increasing from its entry end to its exit end such that the soluble fibre dissolves progressively from the entry end to the exit end,
the progressive dissolution of the fibre creating a microchannel within the gel for directional tissue growth from the entry end to the exit end of the fibre.

The tissue entry end of the soluble fibre may be disposed at a tissue entry end of the gel. In some embodiments, the tissue exit end of the soluble fibre may be disposed at a tissue exit end of the gel, such that the fibre connects the entry and exit ends of the gel. In other embodiments, the tissue exit end of the soluble fibre may be disposed within the scaffold.

In some embodiments a biomaterial for directional tissue growth may comprise;
a gel having tissue entry and tissue exit ends; and,
a soluble fibre within the gel which connects the tissue entry and tissue exit ends of the gel,
wherein the soluble fibre has a cross-sectional area which progressively increases from the entry end to the exit end of the gel.
such that the soluble fibre dissolves progressively from the entry end to the exit end of the gel,
the progressive dissolution of the fibre creating a microchannel for directional tissue growth from the entry end to the exit end of the gel.

A gel comprises a matrix of fibrous material and an interstitial liquid. Gels are formed by the coalescence and elongation of fibrils of the matrix material, as the fibrils form a continuous network around the aqueous interstitial liquid which originally held the monomers. For example, triple helical collagen monomers may be initially dissolved in dilute acid and then induced to polymerise (aggregate) to fibrils (e.g. at 37° and neutral pH). As the fibrils polymerise, there is a phase change and the solid network of fibrils 'supports' the remaining interstitial liquid in approximately the same volume and shape - i.e. it gels. Phase transition from soluble monomer to solid polymer is characteristic of a gel and is important in providing the properties described herein. Gels are distinct from 'sponges', which may be formed from pre-polymerised fibres.

Suitable fibrous, non-woven or sponge matrix materials for use in the gels described herein include naturally occurring polymers, for example proteins, such as silk, fibrin, fibronectin, elastin or collagen (e.g. collagen type I), glycoproteins such as fibronectin, or polysaccharides such as chitin, or cellulose. In some preferred embodiments, the matrix fibres are made collagen. Native fibril forming collagen types are preferred including collagen types are I, II, III, V, VI, IX and XI and combinations of these (e.g. I, III V or II, IX, XI). For example, collagen type I may be used as the scaffold material.

Other suitable fibrous matrix materials include synthetic polymers i.e. polymers that are not naturally present in the human or animal body. Suitable polymers include organic polymers such as polylactone, polyglycone and polycapryolactone and inorganic polymers.

In some embodiments, the fibrous matrix material may be a composite material comprising two or more different types of fibre. For example, the matrix may comprise fibronectin and collagen, collagen and polylactide, fibrin and collagen, or fibrin, collagen and fibronectin.

The interstitial liquid is typically an aqueous liquid which acts as a solvent for the soluble fibre. For example, the liquid may be water with solutes such as salts and proteins dissolved therein. In some embodiments, the interstitial liquid is a cell culture medium suitable for the growth and proliferation of cells.

Techniques for formulating and casting gels for use as biomaterials are well-known in the art.

A soluble fibre is a filament or thread which dissolves in an aqueous environment.

The rate of dissolution of a soluble fibre may be optimised by altering its composition and environment for particular applications.

Soluble fibres may be made of any suitable material including soluble phosphate glass, polycapryolacetone, polyacetate, polyglycolic acid, silks, polysaccarides, or fused or crystallised salts. In some embodiments, phosphate glass fibres are preferred. The solubility rate of a phosphate glass fibre may be optimised in the range from hours to months, by doping the initial glass mixture with ions such as Ca, Alu, Fe, Mn, (J. Knowles et al Biomaterials 22 23 (2001) 3091-3096(6)).

Other suitable soluble fibres include protein fibres which can be degraded by cells or polymer hybrids with connective amino acid chains/peptides. These fibres may be dissolved enzymatically either by extra cellular proteases produced as cells migrate over the fibres or by addition of an exogenous protease, protease activator or limiting coenzyme. For example, eurokinase plasminogen activator may added to a biomaterial to activate endogenous plasminogen in vivo which will degrade (eventually dissolve) fibrin fibres.

A soluble fibre may be incorporated into a gel by any convenient technique. For example, soluble fibres may be inserted between the layers of a gel, added to the gel before casting or may be inserted into the gel after casting. In embodiments comprising more than one cast gel, a soluble fibre may, for example, be sandwiched between the gels. The incorporation of soluble glass fibres into gels is described in Nazhat et al Biomacromolecules 2007 Feb;8(2):543-51.

Preferably, the fibre has a uniform composition which does not vary in different parts of the fibre e.g. longitudinally along the length of the fibre or radially across the cross-section of the fibre. The chemical and physico-chemical properties of the fibre are uniform in different parts of the fibre. A fibre typically has a circular or substantially circular cross-section, although non-circular cross-sections are also possible.

In some embodiments, a soluble fibre may be a single fibre having a cross-sectional area which varies along its length.

In other embodiments, a soluble fibre may be a bundle of fibrils. The number of fibrils in the bundle may vary along its length, so that the cross-sectional area of the bundle of fibrils varies along its length.

A soluble fibre composed of a bundle of fibrils may have increased flexibility relative to surface area.

Soluble fibres suitable for use as described herein have a cross-sectional area which progressively increases from the entry end to the exit end of the gel.

The cross-sectional area may increase continuously from the entry end to the exit end of the gel or may increase in a series of steps from from the entry end to the exit end of the gel.

The fibre may have a cross-sectional area at the entry end of the gel of less than 300 µm², less than 200 µm², less than 100 µm², less than 50 µm² or less than 10 µm². For example, a substantially circular fibre may have a diameter of less than 100 µm, less than 50 µm, less than 20 µm, less than 10 µm or less than 5 µm.

The fibre may have a cross-sectional area at the exit end of the gel of less than 200000 µm², less than 100000 µm², less than 80000 µm², or less than 8000 µm². For example, a substantially circular fibre may have a diameter of less than 500 µm, of less than 300 µm or less than 200 µm.

The rate of dissolution of a soluble fibre in a gel is dependent on the physico-chemical properties of the fibre, the local aqueous chemical environment (e.g. pH, ionic strength, protein concentration, perfusion rate), the surface area: volume ratio of the fibre and the total mass of fibre at a given point along its length. When incorporated into the gel of a biomaterial, the fibre is exposed to a substantially uniform aqueous chemical environment along its length. Furthermore, as described above, the composition of the fibre is also substantially uniform along its length.

The rate of dissolution of the soluble fibre in the gel is therefore determined by the surface area: volume ratio of the fibre, and the total mass of fibre (at a given point). As the cross-section of the fibre increases along its length, the surface area: volume ratio decreases and the mass of the fibre increases, and so the rate of dissolution of the fibre decreases. The fibre therefore dissolves faster at the entry end than the exit end of the gel.

As the fibre dissolves more slowly as its cross-sectional area increases, so dissolution of the fibre will begin at the entry end, where the cross-sectional area is lowest. The fibre will then progressively dissolve in the direction of the exit end, where the cross-sectional area is greatest. In other words, over a period of time, the dissolution front of the fibre (i.e. the boundary of undissolved fibre) moves from the entry end of the gel to the exit end.

Dissolution of the fibre leaves a cavity in the gel in its place. This cavity has approximately the size, shape and position of the original fibre. This cavity forms a microchannel which progressively extends through the gel from the entry end to the exit end as the soluble fibre progressively dissolves.

Initially, tissue can only grow into the microchannel at the entry end of the gel, where the soluble fibre dissolves first. As the microchannel extends through the gel, the tissue can grow further in the direction of the exit end. When the fibre is completely dissolved, the tissue can grow through the exit end of the gel and into the surrounding tissue.

In a biomaterial as described herein, more than one soluble fibre may be disposed within the scaffold.

The number and density of soluble fibres depends on the needs of the tissue and the application. For example, fibres may represent up to about 5%, up to about 10%, up to about 20%, up to about 30%, up to about 40% or up to about 50% of the total cross sectional area of the biomaterial.

The soluble fibres disposed within the scaffold may all possess substantially the same alignment and direct channelling in the same direction or may possess different alignments and direct channelling in different directions.

The alignment of the soluble fibres may differ in different regions of the scaffold. For example, the ends of the scaffold may contain soluble fibres in an anti-parallel alignment to allow tissue entry at both ends. Alternatively, the different zones in the cross section of the scaffold may contain anti-parallel soluble fibres directing tissue growth in opposite directions with the scaffold.

Soluble fibres possessing substantially the same alignment may not be exactly parallel. For example, up to 5%, up to 10% or up to 20% may be angled (i.e. non-parallel) with respect to the majority of the fibres. This may provide a branched plexus of microchannels within the scaffold which mimics a capillary tree.

In some embodiments, the gel may be compacted as described in WO2006/003442 to improve its mechanical properties. Unconfined compaction of a gel expels interstitial liquid, which does not return on removal of the load: i.e. the gel undergoes a plastic compaction. In an untreated gel, the scaffold matrix is generally in a gross, hydrated form. This scaffold structure collapses during plastic compaction without loss of structural detail, dehydrating the scaffold in the gel, and leading to increased density and strength.

Following compaction, the gel may be subjected to repeated cycles of uniaxial tensile loading as described in WO2007/060459 to improve its mechanical properties. Repetitive cycles of loading increase the fusion of collagen fibrils in a compacted collagen gel to produce a biomaterial which has improved material strength (i.e. increased break stress, break strain and/or elastic modulus).

In some embodiments, the scaffold may be seeded with cells, in particular human or other mammalian cells. Cells may confer tissue functionality and provide structures which replace or facilitate the repair of endogenous tissue. For example, the gel may comprise one or more cell types selected from the group consisting of muscle cells to provide contractile structures, vascular and/or neural cells to provide conductive elements, metabolically active secretory cells, such as liver cells, hormone synthesising cells, sebaceous cells, pancreatic islet cells or adrenal cortex cells to provide secretory structures, stem cells, such as bone marrow-derived stem cells, dermal fibroblasts, skin keratinocytes, (and combination layers of the two), Schwann cells for nerve implants, smooth muscle cells and endothelial cells for vessel structures, urothelial and smooth muscle cells for bladder/urethra structures and osteocytes, chondrocytes, and tendon cells for bone and tendon structures.

In some preferred embodiments, the scaffold may be seeded with endothelial cells to line microchannels and facilitate the growth of blood capillaries.

Cells may be distributed interstitially within the gel in any arrangement. For example, the cells may be distributed homogeneously throughout the gel or distributed in defined zones, regions or layers within the gel.

Specific cell types may be positioned at the core of the scaffold to generate attractive or chemo tactic factors which promote tissue in-growth. Suitable stimulatory cell types include smooth muscle and mesenchymal stem cells for promotion of angiogenesis and Schwann cells for promotion of neural in-growth.

Cells may be seeded within the matrix by mixing them with the liquid scaffold matrix and then allowing the liquid matrix to solidify into a gel. Seeding of the matrix is preferably performed under suitable conditions of temperature, pH, ionic strength and sheer to maintain viability, prior to gel formation. The initial cell density in the gel may be from about 1 x 10⁴ to 1 x 10⁷ cells per ml, more preferably from about 5 x 10⁵ to 1 x 10⁶ cells per ml.

The methods described herein allow the production of biomaterials with multiple microchannels which support unidirectional tissue growth.

Biomaterial produced by the present methods may be in any convenient form, for example, it may be a sheet, ring, toroid, capillary, strip, block, tube, particle, or roll.

Another aspect of the invention provides a method of making a biomaterial for directional tissue growth comprising;
incorporating a soluble fibre into a scaffold,
the soluble fibre having a cross-sectional area which progressively increases from an entry end to an exit end of the fibre such that the fibre dissolves progressively from the entry end to the exit end,
the progressive dissolution of the fibre creating a microchannel in the scaffold for directional tissue growth though the scaffold from the entry end to the exit end of the fibre.

In some embodiments, the scaffold may have tissue entry and exit ends which are connected by the soluble fibre. For example, a method of making a biomaterial for unidirectional tissue growth may comprise;
incorporating a soluble fibre into a gel,
wherein the gel has tissue entry and exit ends which are connected by the soluble fibre,
the soluble fibre having a cross-sectional area which progressively increases from the entry end to the exit end of the gel.
such that the fibre dissolves progressively from the entry end to the exit end of the gel,
the progressive dissolution of the fibre creating a microchannel in the gel for directional tissue growth from the entry end to the exit end of the gel.

A soluble fibre having a cross-sectional area which progressively increases along its length which is suitable for incorporation into a gel as described herein may be produced by any suitable technique.

In some embodiments, a suitable fibre may be produced from a standard fibre with a uniform cross-section along its length (i.e. a parallel sided fibre) by a method comprising:
providing a soluble fibre having a uniform cross-section along its length,
exposing the fibre to a solvent which dissolves the fibre,
removing the fibre from the solvent progressively from the second end to the first end,
thereby producing a fibre having a cross-sectional area which progressively increases from a first end to a second end.

The narrow first end of the soluble fibre may, for example, be incorporated into the scaffold at the entry end and the wide second end may be incorporated into the scaffold at the exit end.

In other embodiments, a suitable fibre may be produced by assembling a plurality of soluble fibrils into a bundle. More fibrils may assembled into the bundle of fibrils at the exit than the entry end, so that the cross sectional area is increased at the exit relative to the entry end.

Biomaterials as described herein may be particularly useful in the production of tissue-equivalent implants, in particular in circumstances in which vectored channelling of the tissue growth or re-growth is important.

Vectored channelling may, for example, support the growth or re-growth of neural tissue (i.e. innervation or re-nervation), from the proximal to the distal end of a lesion in nervous tissue from the peripheral or central nervous system, or may be useful in supporting angiogenesis in injured or damaged tissue. Vectored channelling may also be useful where spatial or temporary control of the growth of cells or tissues from the host side of the host-biomaterial interface is required. This may, for example, involve attracting fibroblasts to lay down collagen fibres across the host-biomaterial interface.

The orientation of tissue growth through the implant in all of these applications facilitates biomimetic function for flow perfusion, rapid re-innervation (i.e. towards target) and mechanical strength.

A biomaterial comprising soluble fibres as described herein may be useful as a tissue equivalent implant for the repair or replacement of damaged tissue without additional processing.

Alternatively, additional processing of the biomaterial may be performed to produce a tissue equivalent implant for the repair or replacement of damaged tissue. The biomaterial containing the soluble fibres may, for example, be moulded and/or shaped to produce a tissue equivalent implant. The biomaterial may be moulded into a predetermined shape and/or may be subjected to plastic compaction which may be symmetrical or asymmetrical.

The biomaterial may be shaped, cut or moulded into any convenient implant form, for example, a patch, block, tube, tape, strip, ring, toroid, capillary, roll, sheet or thread. The final shape of the tissue equivalent implant will depend on the particular context in which it is to be used. In some embodiments, the tissue equivalent implant may have a pliable form which is suitable for further shaping.

The tissue equivalent implant is preferably fixable at a site of tissue damage. For example, the implant may be fixable such that the entry end is located adjacent the proximal stump of a damaged tissue and the exit end is located adjacent the distal stump of a damaged tissue. The tissue equivalent implant may be fixed by any convenient technique. For example, it may be sutured or glued in place.

Another aspect of the invention provides a method of treatment of a damaged tissue in an individual comprising;
producing a tissue equivalent implant using a method described herein and,
fixing said implant to said damaged tissue to repair and/or replace said tissue.

Another aspect of the invention provides a method of promoting directional tissue growth and/or repair at a site of tissue damage in an individual comprising
providing a gel having tissue entry and exit ends
the entry and exit ends being linked by a soluble fibre within the gel,
wherein the soluble fibre has a cross-sectional area which progressively increases from the entry end to the exit end,
positioning the gel at a site of tissue growth or repair,
such that the fibre dissolves progressively from the entry end to the exit end to create a microchannel for unidirectional tissue growth from the entry end to the exit end of the gel.

A tissue implant as described herein may be used at a site of tissue damage which comprises a distal and a proximal stump of tissue, for example nervous tissue, which are separated by a lesion or other breakage. The entry end of the gel may be positioned at the distal stump and the exit end at the proximal stump. Tissue growing from the distal stump enters the gel at the entry end, grows along the microchannels formed by dissolution of the soluble fibres and then exits the gel at the exit end adjacent the proximal stump.

Timed growth of neurites through an implant may be useful in spinal repair as ingrowth of inhibitory or blocking tissue (glial scar) into the path of the regenerating neural tissue may be delayed until the regenerating axons have reached the necessary position in the repair (i.e. the exit zone). In this application, the microchannels represent a timed-bridge function which physically bridges the glial scar.

Various further aspects and embodiments of the present invention will be apparent to those skilled in the art in view of the present disclosure.

Certain aspects and embodiments of the invention will now be illustrated by way of example and with reference to the figures and tables described below.
Fig 1 shows mean diameter loss over the length of phosphate based glass fibres after gradual dissolution in 1% Triton in TRIS buffer and subsequent dehydration. Data are expressed as percent ± standard deviation (n=6).
Fig 2 shows reduction in diameter for individual fibre after gradual dissolution in 1%TRITON in TRIS buffer and subsequent dehydration in percent ± standard deviation (n=6).
Fig 3 shows percentage of living Schwann cells in the constructs with and without PGF after 3 days in vitro. N=2 of each type of construct for each time point, 6 fields of view per construct were analysed; data is presented as percentage of live cells ± standard deviation.
Fig 4 shows percentage of living BMSC cells in the constructs with and without PGF after 6 days in vitro. N=2 of each type of construct for each time point, 9 fields of view per construct were analysed; data is presented as percentage of live cells ± standard deviation.

### Experiments

### Methods

### Conical fibres preparation

Dissoluble glass fibres with composition ratio 0.5 (P₂O₅) : 0.25(CaO) : (Na₂ O), average diameter of 30-40 µm (distilled water dissolution time 8-10 hrs; spacing 170µm, were cut at 2.3 x 3.5cm and placed on coverslips and secured using autoclave tape. Coverslips were than placed in small Coplin jars which were than filled with 7 ml of 1% TRITON X-100 (Sigma) in Tris buffer (pH 7.4) solution. Liquid was removed from the vial at the rate of 800µl per hour. Hydrogel build-up was removed by dehydration in ascending alcohols (5 minsx2 for 20, 30, 40, 50, 60 and 90 %, 10 minsx2 in 96% and 15 mins x2 in 100%) finished with triple three minutes wash in HMDS (Hexamethyldisalazane).

### Electron microscopy

Slides were left to dry overnight, than glass fibres were gently removed from the coverslip and mounted on the stubs which were sputter coated following standard procedure. Samples were viewed using scanning electron microscope (Jeol JSM 5500 LV) at the magnification X500. Fibre diameter was measured along the full length every 2 mm. Total number of 6 fibres was measured for the purpose of this report.

The data was than transferred to the spreadsheet and average loss in diameter in µm was calculated.

### Results

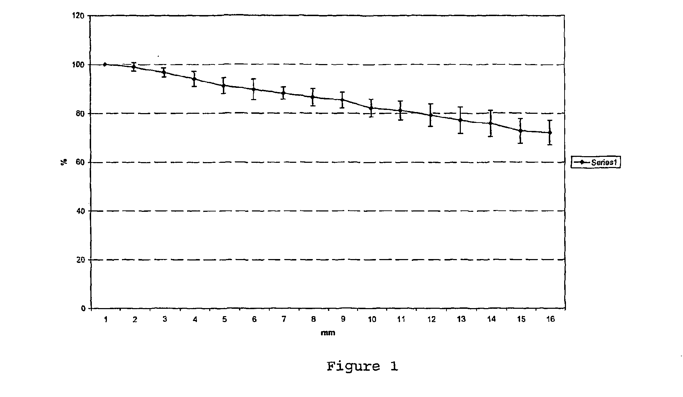

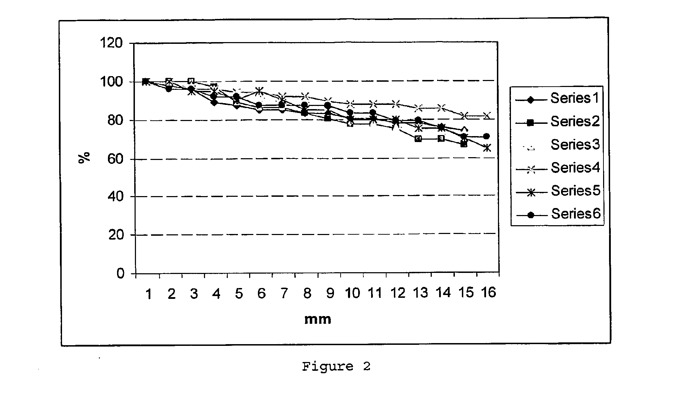

Conical phosphate glass fibres were constructed as described above and examined by EM. The loss in fibre diameter over the full length of fibre after gradual dissolution in 1%Triton™ in TRIS buffer and subsequent dehydration is shown in Figures 1 and 2. Note the mean loss in diameter for 6 fibres was 10.833 ± 5.35 µm over 15.5 mm, giving a mean rate of change 0.7 µm/mm.

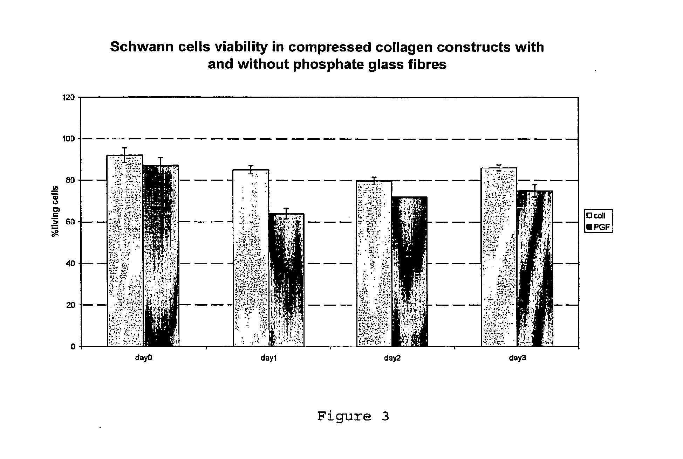

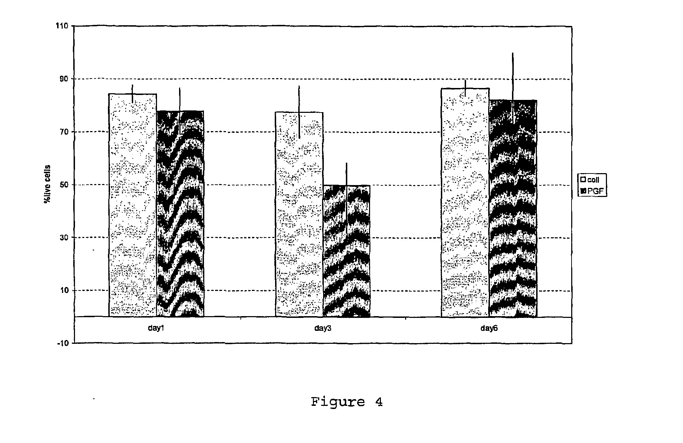

Biocompatibility of the phosphate glass fibres of given composition was tested in compressed collagen constructs for Schwann cells over three days in vitro and Bone Marrow Stromal Cells (BMSC) for day 1, 3 and 6 in vitro. Figures 3 and 4 show the percentage of live cells in the compressed collagen constructs with and without incorporated glass fibres for Schwann cells and BMSC respectively. The lack of Schwann cell death at day 0 suggests that the physical compression process has little damaging effect and reduction in percentage of living cells at day 1 is due to PGF dissolution products. However by day 3 the negative effect is reduced. The fall in viability of BMSCs on day 3 indicates some transient cell damage by PGF dissolution products. The lack of cell death at day 1 indicates that the physical compression process has little damaging effect on these cells.

These results show that the presence of phosphate glass fibres has little effect on the viability of cells in collagen constructs.

## Claims

1. A biomaterial for directional tissue growth comprising;
a soluble fibre disposed within a gel,
the fibre having tissue entry and tissue exit ends, wherein the cross-sectional area of the fibre progressively increasing from its entry end to its exit end such that the soluble fibre dissolves progressively from the entry end to the exit end,
the progressive dissolution of the fibre creating a microchannel within the gel for directional tissue growth from the entry end to the exit end of the fibre.

2. A biomaterial according to claim 1 wherein the tissue entry end of the soluble fibre is disposed at a tissue entry end of the gel and the tissue exit end of the soluble fibre is disposed at a tissue exit end of the gel.

3. A biomaterial according to claim 2wherein the soluble fibre connects the tissue entry and tissue exit ends of the gel and progressive dissolution of the fibre creates a microchannel for directional tissue growth from the entry end to the exit end of the gel.

4. A biomaterial according to any one of the preceding claims wherein the gel is a collagen gel.

5. A biomaterial according to any one of the preceding claims wherein the soluble fibre is a phosphate glass fibre.

6. A biomaterial according to any one of the preceding claims wherein the cross-sectional area of the soluble fibre increases continuously from the entry end to the exit end of the gel.

7. A biomaterial according to any one of claims 1 to 5 wherein the cross-sectional area of the soluble fibre increases in steps from the entry end to the exit end of the gel.

8. A biomaterial according to any one of the preceding claims wherein the gel has undergone plastic compaction and optionally repeated cycles of uniaxial tensile loading following said compaction.

9. A biomaterial according to any one of the preceding claims wherein the gel is seeded with viable cells before plastic compaction.

10. A method of making a biomaterial according to any one of claims 1 to 9 comprising;
incorporating a soluble fibre into a gel,
the soluble fibre having a cross-sectional area which progressively increases from an entry end to an exit end of the fibre such that the fibre dissolves progressively from the entry end to the exit end,
the progressive dissolution of the fibre creating a microchannel in the gel for directional tissue growth though the gel from the entry end to the exit end of the fibre.

11. A method according to claim 10 wherein the soluble fibre is produced by a method comprising:
providing a soluble fibre having a uniform cross-section along its length,
exposing the fibre to a solvent which dissolves the fibre,
removing the fibre from the solvent progressively from the second end to the first end,
thereby producing a fibre having a cross-sectional area which progressively increases from a first end to a second end.

12. A method according to claim 10 wherein the soluble fibre is produced by assembling a plurality of soluble fibrils into a bundle.

13. A tissue equivalent implant comprising a biomaterial according to any one of claims 1 to 9.

14. A tissue equivalent implant according to claim 13 for use in a method of treatment of a damaged tissue in an individual.

## Patentansprüche

1. Biomaterial zum gerichteten Gewebewachstum, umfassend:
eine in einem Gel vorgesehene lösliche Faser,
wobei die Faser Gewebeeintritts- und Gewebeaustrittsenden aufweist,
worin sich die Querschnittsfläche der Faser vom Eintrittsende zum Austrittsende hin zunehmend erhöht, so dass die lösliche Faser sich von Eintrittsende zum Austrittsende hin zunehmend auflöst,
wobei die zunehmende Auflösung der Faser einen Mikrokanal innerhalb des Gels für das gerichtete Gewebewachstum von Eintrittsende zum Austrittsende der Faser erzeugt.

2. Biomaterial nach Anspruch 1, worin das Gewebeeintrittsende der löslichen Faser an einem Gewebeeintrittsende des Gels vorgesehen ist und das Gewebeaustrittsende der löslichen Faser an einem Gewebeaustrittsende des Gels vorgesehen ist.

3. Biomaterial nach Anspruch 2, worin die lösliche Faser das Gewebeeintritts-und das Gewebeaustrittsende des Gels miteinander verbindet und die zunehmende Auflösung der Faser einen Mikrokanal für das gerichtete Gewebewachstum von Eintrittsende zum Austrittsende des Gels erzeugt.

4. Biomaterial nach einem der vorangegangenen Ansprüche, worin das Gel ein Kollagengel ist.

5. Biomaterial nach einem der vorangegangenen Ansprüche, worin die lösliche Faser eine Phosphatglasfaser ist.

6. Biomaterial nach einem der vorangegangenen Ansprüche, worin die Querschnittsfläche der löslichen Faser vom Eintrittsende zum Austrittsende des Gels hin kontinuierlich zunimmt.

7. Biomaterial nach einem der Ansprüche 1 bis 5, worin die Querschnittsfläche der löslichen Faser vom Eintrittsende zum Austrittsende des Gels hin schrittweise zunimmt.

8. Biomaterial nach einem der vorangegangenen Ansprüche, worin das Gel plastische Verdichtung und gegebenenfalls wiederholte Runden einachsiger Zugbeanspruchung im Anschluss an die Verdichtung durchlaufen hat.

9. Biomaterial nach einem der vorangegangenen Ansprüche, worin das Gel vor der plastischen Verdichtung mit Lebendzellen besät wird.

10. Verfahren zur Herstellung einer Biomaterials nach einem der Ansprüche 1 bis 9, umfassend:
das Einarbeiten einer löslichen Faser in ein Gel,
wobei die Faser eine Querschnittsfläche aufweist, die sich vom Eintrittsende zum Austrittsende hin zunehmend erhöht, so dass die lösliche Faser sich von Eintrittsende zum Austrittsende hin zunehmend auflöst,
wobei die zunehmende Auflösung der Faser einen Mikrokanal innerhalb des Gels für das gerichtete Gewebewachstum durch das Gel von Eintrittsende zum Austrittsende der Faser erzeugt.

11. Verfahren nach Anspruch 10, worin die lösliche Faser mittels eines Verfahrens hergestellt wird, das Folgendes umfasst:
das Bereitstellen einer löslichen Faser mit gleichmäßigem Querschnitt in Längsrichtung,
das Aussetzen der Faser gegenüber einem Lösungsmittel, das die Faser auflöst,
das vom zweiten Ende zum ersten Ende verlaufende Entfernen der Faser aus dem Lösungsmittel,
wodurch eine Faser mit einer sich von einem ersten Ende zu einem zweiten Ende hin zunehmenden erhöhenden Querschnittsfläche produziert wird.

12. Verfahren nach Anspruch 10, worin die lösliche Faser durch Zusammenfassung einer Vielzahl von löslichen Fäserchen zu einem Bündel hergestellt wird.

13. Gewebeäquivalentes Implantat, umfassend ein Biomaterial nach einem der Ansprüche 1 bis 9.

14. Gewebeäquivalentes Implantat nach Anspruch 13 zur Verwendung in einem Verfahren zur Behandlung eines beschädigten Gewebes bei einem Individuum.

## Revendications

1. Biomatériau pour une croissance de tissu directionnelle comprenant:
une fibre soluble disposée dans un gel, la fibre ayant des extrémités d'entrée de tissu et de sortie de tissu,
où la zone en section transversale de la fibre augmente progressivement de son extrémité d'entrée à son extrémité de sortie de sorte que la fibre soluble se dissous progressivement de l'extrémité d'entrée à l'extrémité de sortie, la dissolution progressive de la fibre créant un microcanal dans le gel pour une croissance de tissu directionnelle de l'extrémité d'entrée à l'extrémité de sortie de la fibre.

2. Biomatériau selon la revendication 1, où l'extrémité d'entrée du tissu de la fibre soluble est disposée à l'extrémité d'entrée du tissu du gel, et l'extrémité de sortie du tissu de la fibre soluble est disposée à l'extrémité de sortie du tissu du gel.

3. Biomatériau selon la revendication 2, où la fibre soluble relie les extrémités d'entrée de tissu et de sortie de tissu du gel, et la dissolution progressive de la fibre crée un microcanal pour la croissance tissulaire directionnelle de l'extrémité d'entrée à l'extrémité de sortie du gel.

4. Biomatériau selon l'une quelconque des revendications précédentes, où le gel est un gel de collagène.

5. Biomatériau selon l'une quelconque des revendications précédentes, où la fibre soluble est une fibre de verre de phosphate.

6. Biomatériau selon l'une quelconque des revendications précédentes, où la zone en section transversale de la fibre soluble augmente continuellement de l'extrémité d'entrée à l'extrémité de sortie du gel.

7. Biomatériau selon l'une quelconque des revendications 1 à 5, où la zone en section transversale de la fibre soluble augmente par étapes de l'extrémité d'entrée à l'extrémité de sortie du gel.

8. Biomatériau selon l'une quelconque des revendications précédentes, où le gel a subi un compactage plastique et des cycles répétés optionnellement de charges de traction uniaxiales à la suite dudit compactage.

9. Biomatériau selon l'une quelconque des revendications précédentes, où le gel est ensemencé avec des cellules viables avant le compactage plastique.

10. Méthode de fabrication d'un biomatériau selon l'une quelconque des revendications 1 à 9, comprenant: incorporer une fibre soluble dans un gel, la fibre soluble ayant une zone en section transversale qui augmente progressivement d'une extrémité d'entrée à une extrémité de sortie de la fibre de telle sorte que la fibre se dissous progressivement de l'extrémité d'entrée à l'extrémité de sortie, la dissolution progressive de la fibre créant un microcanal dans le gel pour la croissance tissulaire directionnelle à travers le gel de l'extrémité d'entrée à l'extrémité de sortie de la fibre.

11. Méthode selon la revendication 10, où la fibre soluble est produite par une méthode comprenant:
réaliser une fibre soluble ayant une section transversale uniforme sur sa longueur, exposer la fibre à un solvant qui dissous la fibre,
retirer la fibre du solvant progressivement de la seconde extrémité à la première extrémité, en produisant ainsi une fibre ayant une zone en section transversale qui augmente progressivement d'une première extrémité à une seconde extrémité.

12. Méthode selon la revendication 10, dans laquelle la fibre soluble est produite en assemblant plusieurs fibrilles solubles en un faisceau.

13. Implant équivalent à du tissu comprenant un biomatériau selon l'une quelconque des revendications 1 à 9.

14. Implant équivalent à du tissu selon la revendication 13 pour utilisation dans une méthode de traitement d'un tissu endommagé chez un individu.
